(19) Europäisches Patentamt
European Patent Office
Office européen des brevets

(11) Veröffentlichungsnummer: **0 251 115 B1**

(12) # EUROPÄISCHE PATENTSCHRIFT

(45) Veröffentlichungstag der Patentschrift: **21.08.91**

(51) Int. Cl.⁵: **C07C 45/49**, C07C 205/44, C07C 49/80, C07C 47/232

(21) Anmeldenummer: **87108945.4**

(22) Anmeldetag: **23.06.87**

(54) **Verfahren zur Herstellung von beta-Halogenvinyl- und beta,beta-Dihalogenethylarylketonen.**

(30) Priorität: **03.07.86 DE 3622296**

(43) Veröffentlichungstag der Anmeldung:
**07.01.88 Patentblatt 88/01**

(45) Bekanntmachung des Hinweises auf die Patenterteilung:
**21.08.91 Patentblatt 91/34**

(84) Benannte Vertragsstaaten:
**BE DE FR GB IT NL**

(56) Entgegenhaltungen:
**EP-A- 0 051 386**
**FR-A- 927 473**
**US-A- 3 094 561**
**DE-C- 642 147**
**FR-M- 8 333**
**US-A- 4 378 288**

(73) Patentinhaber: **BASF Aktiengesellschaft**
**Carl-Bosch-Strasse 38**
**W-6700 Ludwigshafen(DE)**

(72) Erfinder: **Goetz, Norbert, Dr.**
**Schoefferstrasse 25**
**W-6520 Worms 1(DE)**
Erfinder: **Wild, Jochen, Dr.**
**An der Marlach 7**
**W-6705 Deidesheim(DE)**

EP 0 251 115 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein verbessertes Verfahren zur Herstellung von β-Halogenvinyl- und β,β-Dihalogenethylarylketonen der allgemeinen Formeln Ia und Ib

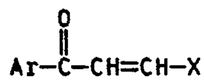

(Ia)

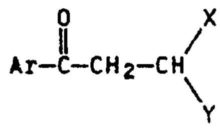

(Ib)

in denen Ar einen Arylrest und X und Y Chlor oder Brom bedeuten, durch Umsetzung eines Arylcarbonsäurehalogenids II

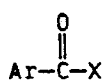

(II)

mit Acetylen bzw. einem Vinylhalogenid III

$CH_2=CH\text{-}Y$ (III)

mittels einer Lewis-Säure.

Außerdem betrifft die Erfindung ein neues Verfahren zur Herstellung von Arylacroleinen IV

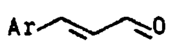

(IV)

sowie neue Verbindungen aus dieser Klasse.

Aus der CH-PS 249 067 ist die Umsetzung von Arylcarbonsäurechloriden mit Vinylchlorid in Tetrachlorkohlenstoff in Gegenwart von Aluminiumchlorid zu den Aryl-chlorvinylketonen des Typs Ia bekannt.

Aus J.Gen.Chem. of USSR 26, 643 (1956) ist die Umsetzung von Arylcarbonsäurechloriden mit Acetylen in Dichlorethan in Gegenwart von Aluminiumchlorid zu den Aryl-β-chlorvinylketonen des Typs Ia bekannt. Zur Herabsetzung der Teerbildung (Verharzung) wurde die Reaktionszeit auf ca. 6 bis 7 Stunden verkürzt. Dies ermöglicht eine leichtere Isolierung des Produktes, ist aber mit dem Nachteil geringeren Umsatzes und niedrigerer Ausbeuten, die etwa bis zu 60 % betragen, verbunden.

Aus Chemical Reviews 66, 161ff (1966) und Houben-Weyl, Bd. 7/2a, S. 480ff sind weitere nach diesen Methoden hergestellte Verbindungen des Typs Ia bekannt.

Die wichtigsten bislang bekannten Herstellungsmethoden für die Arylacroleine IV (Zimtaldehydderivate) gehen von Benzaldehydderivaten aus, an die ein $C_2$-Baustein addiert wird.

Aus Organic Reactions 16, 103 (1968) ist die direkte Aldolkondensation von Acetaldehyd an Benzaldehyde bekannt, jedoch werden nur in wenigen Fällen reine Produkte in befriedigenden Ausbeuten erhalten. Aufwendige Destillationsschritte sind notwendig zur Abtrennung der Selbstkondensationsprodukte des Acetaldehyds und vinyloger Zimtaldehyde, die sich durch weitere Anlagerung von Acetaldehyd an Zimtaldehyd bilden.

Weiterhin ist aus Houben-Weyl, Bd. 7/1, Seite 116ff bekannt, daß Vinylether in Gegenwart von Lewis-Säuren an Benzaldehyde oder besonders an deren Acetale addiert werden können. Hinderlich für eine breite Anwendung der Methode ist die große Polymerisationsneigung des Vinylethers selbst, die weitere Addition des Vinylethers an das entstehende Produkt und vor allem die Reaktionsträgheit der Benzaldehydacetale.

Der Erfindung lag daher die Aufgabe zugrunde, Arylacroleine IV, die wichtige Zwischenverbindungen in Wirkstoffsynthesen sind, besser zugänglich zu machen, insbesondere die für den Syntheseweg zu den Arylacroleinen IV dienenden β-Halogenvinyl- und β,β-Dihalogenethyl-arylketone Ia und Ib auf einfachere Weise herzustellen.

Demgemäß wurde ein verbessertes Verfahren zur Herstellung von β-Halogenvinyl-und β,β-Dihalogenethyl-arylketonen der allgemeinen Formeln Ia und Ib

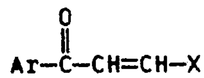

(Ia)

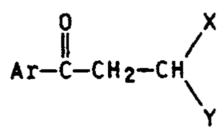

(Ib)

in denen Ar einen Arylrest und X und Y Chlor oder Brom bedeuten, durch Umsetzung eines Arylcarbonsäurehalogenids II

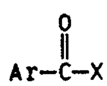

(II)

mit Acetylen bzw. einem Vinylhalogenid III

$CH_2 = CH\text{-}Y$ (III)

mittels einer Lewis-Säure gefunden, das dadurch gekennzeichnet ist, daß man die Umsetzung in Gegenwart von $10^{-4}$ bis 30 mol%, bezogen auf II, eines Radikalinhibitors vornimmt.

Außerdem wurde ein Verfahren zur Herstellung von Arylacroleinen der allgemeinen Formel IV

$Ar\text{-}CH = CH\text{-}CHO$ (IV)

gefunden, welches dadurch gekennzeichnet ist, daß man die Verbindungen Ia und Ib

a) in einer Lösung eines $C_1$-$C_4$-Alkanols mit einem Alkali- oder Erdalkalimetall-$C_1$-$C_4$-alkoholat zu den Acetalen V

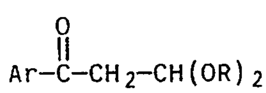

(V)

R = $C_1$-$C_4$-Alkyl

umsetzt,

b) die Verbindungen V zu den Alkoholen VI

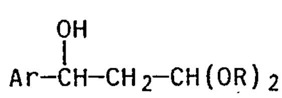

(VI)

hydriert, und

c) die Verbindungen VI ohne Abtrennung des Lösungsmittels mit einer Säure unter Acetalspaltung und

Dehydratisierung in die Verbindungen IV überführt.

Ferner wurden neue Verbindungen aus der Gruppe der Zimtaldehyde IV gefunden.

Als Ausgangsverbindungen II eignen sich besonders solche, in denen x für Chlor oder Brom steht. Unter den Arylgruppen kommen vornehmlich die Phenylgruppe und die $\alpha$- und $\beta$-Naphthylgruppe in Betracht, die ihrerseits durch $C_1$-$C_8$-Alkyl- und $C_1$-$C_8$-Alkoxygruppen, Nitrogruppen, Cyanogruppen und Halogen wie Fluor, Chlor und Brom substituiert sein können.

Besondere Bedeutung haben hierbei Halogenphenyl- und Nitrophenylgruppen sowie gemeinsam durch Halogen- und Nitrogruppen substituierte Phenylgruppen gemäß der allgemeinen Formel

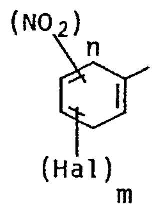

in der Hal gleiche oder verschiedene Substituenten aus der Gruppe von Fluor, Chlor und Brom bedeutet, m für eine Zahl von 0 bis 5 und n für eine Zahl von 0 bis 2 steht, wobei m + n > 0 ist. Die entsprechenden Ausgangsverbindungen sind größtenteils bekannt oder nach bekannten Methoden erhältlich.

Die Reaktionsschritte der erfindungsgemäßen Verfahren zur Herstellung von Ia und Ib bzw. von V seien durch das folgende Schema verdeutlicht:

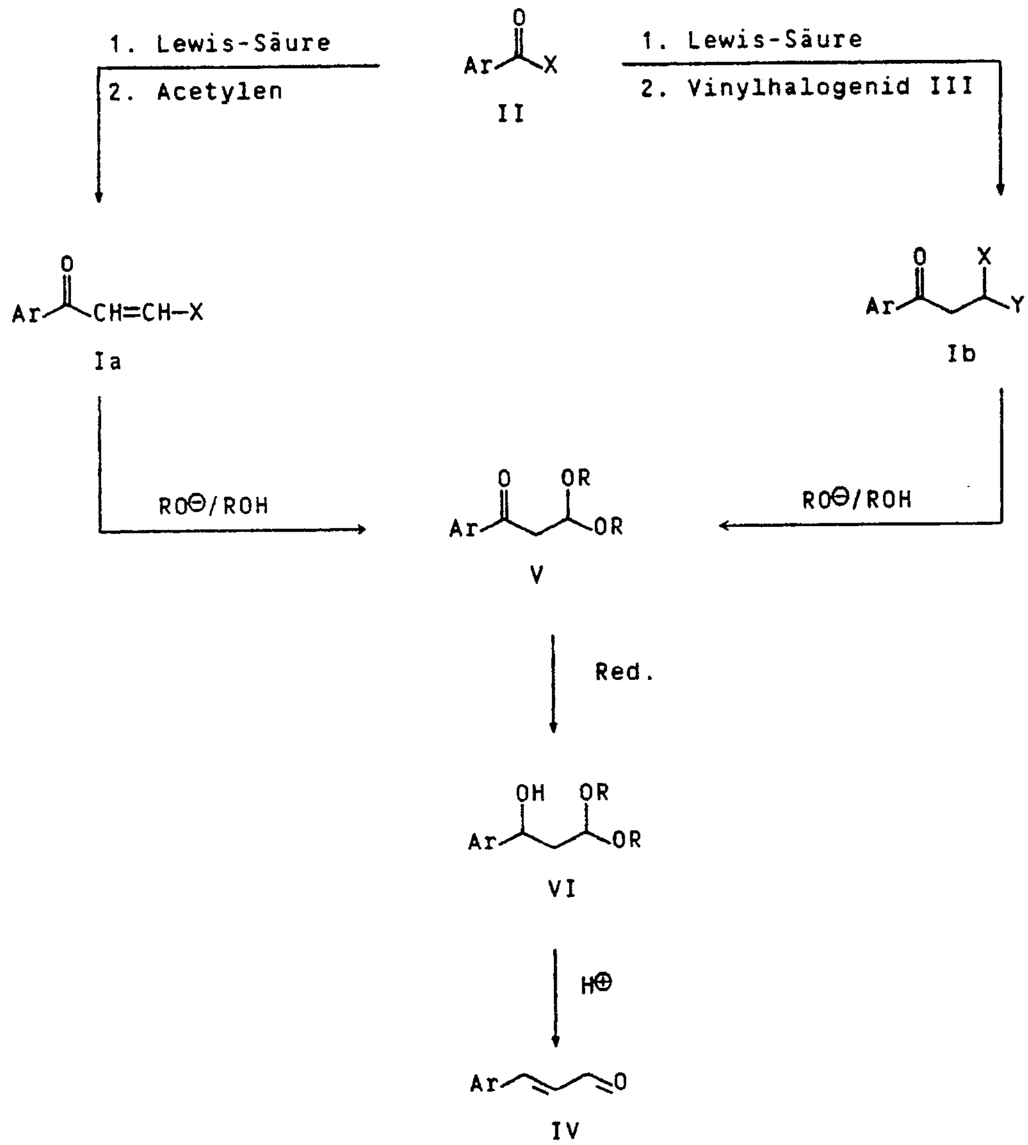


Man kann die Verbindungen II entweder mit Acetylen oder mit einem Vinylhalogenid III, wobei Vinylchlorid bevorzugt wird, zu Ia bzw. Ib umsetzen, wobei prinzipiell die gleichen Verfahrensbedingungen in Betracht kommen.

Als Lewis-Säure ist Aluminiumchlorid bevorzugt. Es eignen sich außerdem beispielsweise Eisen-(III)-chlorid, Zinkchlorid, Antimonpentachlorid, Zinn-(IV)-chlorid, Titantetrachlorid, Bortrifluorid oder Bortrifluoridaddukte mit Diethyl- oder Dimethylether, Phosphorsäure oder Essigsäure. Die Umsetzung läßt sich mit 1 bis 5 Äquivalenten der Lewis-Säure, besonders bevorzugt mit 1 bis 1,5 Äquivalenten, bezogen auf II, durchführen.

Vorzugsweise nimmt man die Umsetzung in Gegenwart eines inerten Lösungsmittels, z.B. in chlorierten Kohlenwasserstoffen wie Methylenchlorid, Chlorbenzol, Tetrachlorkohlenstoff, Dichlorethan, Trichlorethan oder in Nitroverbindungen wie Nitrobenzol oder Nitromethan vor, dessen Menge so bemessen sein sollte, daß die Reaktionspartner eine homogene Lösung bilden. Die hierzu erforderlichen Mindestmengen, die unschwer zu ermitteln sind, liegen in der Regel bei etwa 0,5 bis 20 kg pro kg Verbindung II. Größere Mengen sind möglich, bringen meist aber keine merklichen Vorteile mehr.

Zweckmäßigerweise legt man die Ausgangsverbindung II vor und leitet das Acetylen bzw. das Vinylhalogenid (III) ein, wobei sich die Verfahrensweise wie üblich kontinuierlich gestalten läßt.

Die Reaktion verläuft ab etwa -10 °C mit merklicher Geschwindigkeit. Oberhalb von 80 °C nimmt die Reaktionsgeschwindigkeit nicht mehr nennenswert zu, und es ist mit der Bildung von Nebenprodukten zu rechnen. Im allgemeinen empfehlen sich Reaktionstemperaturen zwischen 0 und 70 °C.

Man kann die Umsetzung bei Normaldruck, vermindertem oder leicht erhöhtem Druck - etwa bis zu 10

bar - vornehmen. Werden wie im Falle der Verwendung von Friedel-Crafts-Katalysatoren als Lewis-Säuren Halogenwasserstoffe abgespalten, so kann sich zu deren leichteren Entfernung besonders das Arbeiten unter vermindertem Druck - etwa hinab bis zu 100 mbar - empfehlen.

Das erfindungswesentliche Merkmal für die Umsetzung von II zu Ia bzw. Ib ist die Mitverwendung eines Radikalinhibitors in Mengen von $10^{-4}$ bis 30 mol%, vorzugsweise 0,01 bis 2 mol%, bezogen auf die Verbindung II. Als Radikalinhibitoren eignen sich Verbindungen verschiedener chemischer Konstitution, z.B. die von Phenol-Typ wie Hydrochinon, Pyrogallol, p-tert.-Butylbrenzkatechin, 2,4-Dichlor-6-nitrophenol oder die vom Chinon-Typ wie Benzochinon.

Bevorzugte Verbindungen dieser Art sind Hydrochinon und Benzochinon.

Nach beendeter Umsetzung kann man wie üblich auf Ia und Ib aufarbeiten, etwa durch Eintragen der Reaktionsmischung in Eiswasser und anschließende Extraktion. Die weitere Reinigung kann durch Destillation oder durch Extraktion des Rohproduktes mit heißem Hexan erfolgen.

Zur Herstellung der Arylacroleine IV versetzt man Ia bzw. Ib mit einem Alkoholat ROM (M = Alkali- oder Erdalkalimetall) bei Temperaturen von -30 bis +20° C, vorzugsweise bei 0 bis -20° C in alkoholischer Lösung.

Die hierbei erhältlichen Acetale V werden wie üblich zu den Hydroxyverbindungen VI hydriert, z.B. mit Hydridreduktionsmitteln wie beispielsweise Natriumborhydrid bei 0 bis 50° C oder durch katalytische Hydrierung mit Pt-, Pd-, Ru-, Rh-, Ni-, Co- oder Cu-Katalysatoren bei 0 bis 200° C und 1 bis 100 bar.

Die Dehydratisierung zu den Arylacroleinen IV kann unter sauren Bedingungen beispielsweise mit Mineralsäuren wie Salzsäure, Schwefelsäure, Phosphorsäure, Perchlorsäure oder organischen Säuren wie Ameisensäure und Oxalsäure, bei Temperaturen von 20 bis 120° C, vorzugsweise 80 bis 100° C erfolgen.

Obwohl bei der erfindungsgemäßen Herstellung der Arylacroleine der allgemeinen Formel IV auf eine Reinigung und Isolierung der Zwischenprodukte weitgehend verzichtet werden kann, ist es natürlich möglich, die Produkte Ia, Ib, V und VI zu isolieren.

Als Beispiele für Benzoesäurehalogenidderivate II seien genannt:
2-Fluorbenzoesäurechlorid
3-Fluorbenzoesäurechlorid
4-Fluorbenzoesäurechlorid
2-Chlorbenzoesäurechlorid
3-Chlorbenzoesäurechlorid
4-Chlorbenzoesäurechlorid
2,4-Dichlorbenzoesäurechlorid
3,5-Dichlorbenzoesäurechlorid
2,6-Difluorbenzoesäurechlorid
2-Chlor-5-nitrobenzoesäurechlorid

Das erfindungsgemäße Verfahren zur Herstellung der β-Halogenvinyl- und β,β-Dihalogenethyl-arylketone Ia und Ib zeichnet sich durch einen glatten Reaktionsablauf ohne das Auftreten von Polymeren (Teerbildung, Verharzung) und der daraus resultierenden beträchtlichen Ausbeutensteigerung durch die Mitverwendung eines Radikalinhibitors aus.

Der Vorteil des erfindungsgemäßen Verfahrens zur Herstellung von Arylacroleinen IV gegenüber den bisher bekannten Synthesemethoden besteht zum einen darin, daß als Ausgangsstoffe die leicht zugänglichen Benzoesäuren bzw. deren Säurehalogenide und nicht die schwerer herzustellenden, kostspieligen Benzaldehyde eingesetzt werden. Ferner zeichnet sich das Verfahren durch hohe Ausbeuten und einfache Verfahrensschritte über alle Stufen hinweg aus, weshalb es sich besonders für den technischen Maßstab eignet.

Die Arylacroleinderivate IV stellen wichtige Zwischenprodukte für die Herstellung von Riechstoffen, Pharmazeutika und Agrarchemikalien dar.

<u>Beispiel 1</u>

Herstellung von Verbindungen Ia mit Acetylen

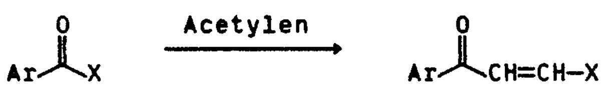


II Ia

7,9 mol eines Benzoesäurehalogenids II und 5 g Hydrochinon (45,4 mmol) in 1,7 l Methylenchlorid werden bei -20 °C mit 137 g Aluminiumchlorid versetzt und 20 Min. gerührt. Bei 40 bis 50 °C wurde Acetylen bis zum Ende der Gasaufnahme eingeleitet (ca. 4 h). Danach wird die Mischung in Eiswasser eingerührt, die organische Phase abgetrennt, getrocknet und eingeengt. Der Rückstand wird entweder 3mal mit heißem Hexan extrahiert oder destillativ aufgearbeitet.

| Ar | X | Fp [°C] | Kp [°C]/mbar | Ausbeute |
|---|---|---|---|---|
| 2,4-Dichlorphenyl | Cl | 63-67 | | 92 % |
| 3,5-Dichlorphenyl | Cl | ölige Masse | | 96 % |
| 2-Chlorphenyl | Cl | | 95/0.3 | 82 % |
| 2,6-Difluorphenyl | Cl | | 112/0.3 | 93 % |
| 2-Fluorphenyl | Cl | | 122/2.5 | 72 % |
| 4-Fluorphenyl | Cl | | 130/2.4 | 99 % |
| 2-Chlor-5-nitrophenyl | Cl | 72-74 | | 95 % |

<u>Beispiel 2</u>

Herstellung von β-Ketoacetalen V

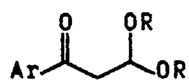

(V)

3,35 mol eines β-Halogenvinylketons Ia oder eines β,β-Dihalogenethylketons Ib, gelöst in 2,8 l Methanol, werden bei -15 °C mit 560 g einer 30 %igen Natriummethylat versetzt und 80 Min. gerührt. Dabei läßt man die Temperatur auf 20 °C ansteigen. Unter guter Durchmischung wird die Lösung in 1,2 l Wasser gegossen. Dadurch wird 1 l Methylenchlorid zugegeben und anschließend werden die Phasen getrennt. Trocknen und Abtrennen des Lösungsmittels ergibt reines β-Ketoacetal V.

| Ar | R | Ausbeute |
|---|---|---|
| 2,4-Dichlorphenyl | $CH_3$ | 95 % |
| 3,5-Dichlorphenyl | $CH_3$ | 92 % |
| 2-Chlorphenyl | $CH_3$ | 94 % |
| 2,6-Difluorphenyl | $CH_3$ | 96 % |
| 2-Fluorphenyl | $CH_3$ | 100 % |
| 4-Fluorphenyl | $CH_3$ | 97 % |
| 2-Chlor-5-nitrophenyl | $CH_3$ | 99 % |

<u>Beispiel 3</u>

Herstellung von β-Ketoacetalen V ausgehend von Benzoesäurehalogeniden und Vinylhalogeniden

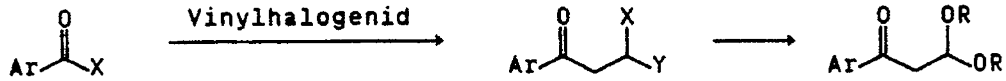


II Ib V

In eine Mischung aus 7,9 mol Benzoesäurehalogenid II, 5 g (45,4 mmol) Hydrochinon, 137 g Aluminiumchlorid und 1,7 l Methylenchlorid wird bei 40 bis 50 °C Vinylchlorid bis zum Ende der Gasaufnahme eingeleitet. Die Mischung wird in Eiswasser eingerührt, die organische Phase getrocknet und eingeengt. Der Rückstand wird in 3 l Methanol gelöst und bei - 10 °C mit 1,4 kg einer 30 %igen Natriummethylatlösung versetzt und 80 min gerührt. Dabei läßt man die Mischung auf Raumtemperatur erwärmen. Nach der üblichen Aufarbeitung wird der Rückstand destilliert.

| Ar | R | Ausbeute |
|---|---|---|
| 2-Chlorphenyl | $CH_3$ | 75 % |
| 2,6-Difluorphenyl | $CH_3$ | 78 % |
| 4-Fluorphenyl | $CH_3$ | 81 % |

Beispiel 4

Herstellung von β-Hydroxyacetalen VI

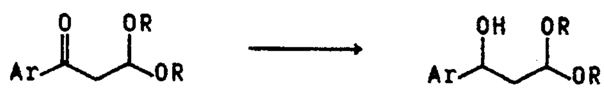

V VI

2,1 mol eines β-Ketoacetal V in 2 l Methanol werden bei 5 °C portionsweise mit 98 g $NaBH_4$ versetzt. Nach beendeter Zugabe wird noch 2 h nachgerührt, wonach nacheinander 1 l Wasser und 1 l Methylenchlorid zugegeben wird. Die organische Phase wird abgetrennt, getrocknet und eingeengt. Das Rohprodukt wird ohne weitere Reinigung zum Arylacrolein gespalten (Beispiel 5).

Beispiel 5

Herstellung von Arylacroleinen IV

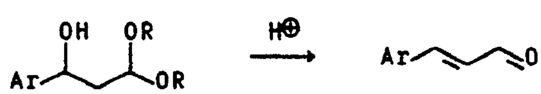


VI IV

2,1 mol eines rohen β-Hydroxyacetal VI aus Beispiel 4 werden langsam in 0,9 l Ameisensäure erwärmt. Dabei werden leichtflüchtige Bestandteile abdestilliert, bis eine Sumpftemperatur von 100 °C erreicht war. Der Rückstand wird einer fraktionierenden Destillation unterworfen.

| Ar | Fp [°C] | Kp [°C]/mbar | Ausbeute |
|---|---|---|---|
| 2,4-Dichlorphenyl | 102-104 | | 79 % |
| 3,5-Dichlorphenyl | 110-111 | | 86 % |
| 2-Chlorphenyl | 55- 56 | | 93 % |
| 2,6-Difluorphenyl | 42,5-44,5 | | 85 % |
| 2-Fluorphenyl | | 86/0,6 | 89 % |
| 4-Fluorphenyl | | 103/0,01 | 91 % |
| 2-Chlor-5-nitrophenyl | 121-123 | | 46 % |

## Patentansprüche

1. Verfahren zur Herstellung von β-Halogenvinyl- und β,β-Dihalogenethyl-arylketonen der allgemeinen Formeln Ia und Ib

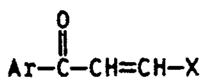
(Ia)

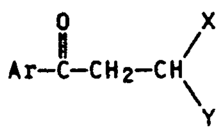
(Ib)

in denen Ar einen Arylrest und X und Y Chlor oder Brom bedeuten, durch Umsetzung eines Arylcarbonsäurehalogenids II

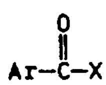
(II)

mit Acetylen bzw. einem Vinylhalogenid III

$CH_2=CH\text{-}Y$ (III)

mittels einer Lewis-Säure, dadurch gekennzeichnet, daß man die Umsetzung in Gegenwart von $10^{-4}$ bis 30 mol%, bezogen auf II, eines Radikalinhibitors vornimmt.

2. Verfahren nach Anspruch 1, dadurch gekennzeichnet, daß man als Radikalinhibitor Hydrochinon, Pyrogallol, p-tert.-Butylbrenzcatechin, Benzochinon oder 2,4-Dichlor-6-nitrophenol verwendet.

3. Verfahren nach den Ansprüchen 1 und 2, dadurch gekennzeichnet, daß man zur Herstellung der Verbindungen der allgemeinen Formeln Ia' und Ib'

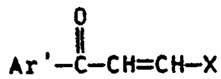

(Ia')

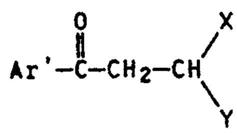

(Ib'),

in denen Ar' einen Rest

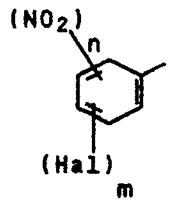

bedeutet, in dem Hal gleiche oder verschiedene Substituenten aus der Gruppe von Fluor, Chlor und Brom bedeutet, m für eine Zahl von 0 bis 5 und n für eine Zahl von 0 bis 2 steht, wobei $m + n > 0$ ist, von den Verbindungen II'

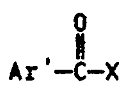

(II')

ausgeht.

4. Verfahren zur Herstellung von Arylacroleinen der allgemeinen Formel IV

Ar-CH = CH-CHO (IV)

dadurch gekennzeichnet, daß man die nach den Ansprüchen 1 bis 3 hergestellten Verbindungen Ia und Ib

a) in einer Lösung eines $C_1$-$C_4$-Alkanols mit einem Alkali- oder Erdalkalimetall-$C_1$-$C_4$-alkoholat zu den Acetalen V

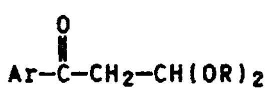

(V)

R = $C_1$-$C_4$-Alkyl

umsetzt.

b) die Verbindungen V zu den Alkoholen VI

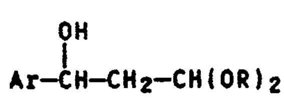

(VI)

hydriert, und
c) die Verbindungen VI ohne Abtrennung des Lösungsmittels mit einer Säure unter Acetalspaltung und Dehydratisierung in die Verbindungen IV überführt.

## Claims

1. A process for preparing a $\beta$-halovinyl or $\beta,\beta$-dihaloethyl aryl ketone of the formula Ia or Ib

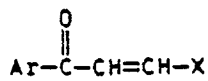

(Ia)

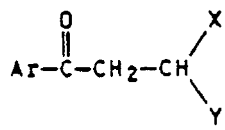

(Ib)

where Ar is aryl and X and Y are each chlorine or bromine, by reacting an arylcarbonyl halide II

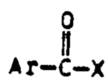

(II)

with acetylene or with a vinyl halide III

$CH_2=CH-Y$ (III)

using a Lewis acid, which comprises carrying out the reaction in the presence of $10^{-4}$ to 30 mol%, based on II, of a radical inhibitor.

2. A process as claimed in claim 1, wherein hydroquinone, pyrogallol, p-tert-butylcatechol, benzoquinone or 2,4-dichloro-6-nitrophenol is used as radical inhibitor.

3. A process as claimed in claims 1 and 2, wherein the starting material used for preparing a compound of the formula Ia' or Ib'

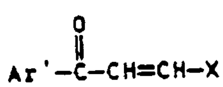

(Ia')

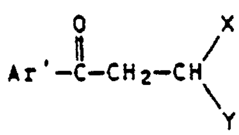

(Ib')

where Ar' is

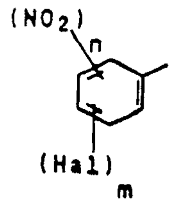

where Hal is identical or different substituents from the group comprising fluorine, chlorine and bromine, m is from 0 to 5 and n is from 0 to 2, where m + n > 0, is a compound II'

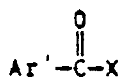

(II').

4. A process for preparing an arylacrolein of the formula IV

Ar-CH = CH-CHO (IV)

which comprises reacting a compound Ia or Ib prepared as claimed in claims 1 to 3

a) in a solution of a $C_1$-$C_4$-alkanol with an alkali metal or alkaline earth metal $C_1$-$C_4$-alcoholate to give the acetal V

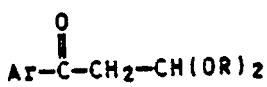

(V)

R = $C_1$-$C_4$-alkyl

b) hydrogenating the compound V to give the alcohol VI

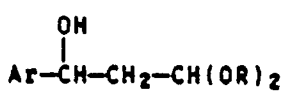

(VI)

and

c) converting the compound VI, without removing the solvent, with an acid with acetal cleavage and dehydration into the compound IV.

## Revendications

1. Procédé de préparation de $\beta$-halogénovinyl- et $\beta,\beta$-dihalogénoéthyl-arylcétones de formules générales Ia et Ib

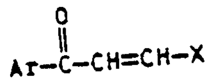

(Ia)

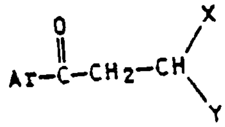

(Ib)

dans lesquelles Ar représente un reste aryle et X et Y représentent le chlore ou le brome, par réaction d'une halogénure d'acide arylcarboxylique II

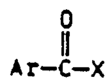

(II)

avec l'acétylène ou un halogénure de vinyle III

$CH_2 = CH\text{-}Y$ (III)

à l'aide d'un acide de Lewis, caractérisé en ce qu'on mène la réaction en présence de $10^{-4}$ à 30% en moles, par rapport à II, d'un inhibiteur de radicaux.

2. Procédé selon la revendication 1, caractérisé en ce qu'on utilise comme inhibiteur de radicaux l'hydroquinone, le pyrogallol, la p-tert.-butylbenzocatéchine, la benzoquinone ou le 2,4-dichloro-6-nitrophénol.

3. Procédé selon l'une ou l'autre des revendications 1 et 2, caractérisé en ce que, pour la préparation de composés de formules générales Ia' et Ib'

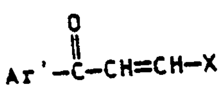

(Ia')

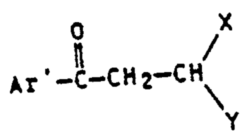

(Ib').

dans lesquelles Ar' représente un reste

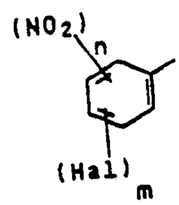

où Hal représente des substituants identiques ou différents du groupe du fluor, du chlore et du brome, m est mis pour un nombre de 0 à 5 et n pour un nombre de 0 à 2, avec m + n > 0,
on part des composés II'

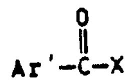

(II')

4. Procédé de préparation d'arylacroléines de formule générale IV

Ar-CH=CHO (IV)

caractérisé en ce que

a) on fait réagir les composés Ia et Ib obtenus selon les revendications 1 à 3, dans une solution d'un alcanol en $C_1$-$C_4$, avec un alcoolate de métal alcalin ou alcalino-terreux pour former les acétals V

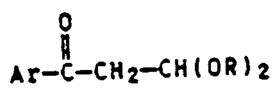

(V)

R = alkyle en $C_1$-$C_4$

b) on hydrate les acétals V en alcools VI

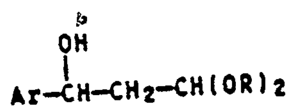

(VI)

et

c) on transforme les composés VI en composés IV, sans chasser le solvant, avec un acide par coupure de l'acétal et déshydratation.